# EUROPEAN PATENT APPLICATION

(11) **EP 4 779 009 A1**
(43) Date of publication of application: **22.07.2026**
(21) Application number: 24865001.2
(22) Date of filing: 24.05.2024
(51) Int. Cl.: C12M 1/00

(54) **PROCESS DEVELOPMENT ASSISTANCE SYSTEM AND PROCESS DEVELOPMENT ASSISTANCE METHOD**

(30) Priority: 11.09.2023 JP 2023147233
(71) Applicant: Hitachi, Ltd., Tokyo 100-8280 (JP)
(72) Inventor: KIMURA Yuya, Tokyo 100-8280 (JP); SHIBUYA Keisuke, Tokyo 100-8280 (JP); NOTO Kazuhiko, Tokyo 100-8280 (JP); TSUBOI Taiki, Tokyo 100-8280 (JP); KATO Hiroaki, Tokyo 100-8280 (JP); SHINOZUKA Ayano, Tokyo 100-8280 (JP)
(74) Representative: MERH-IP Matias Erny Reichl Hoffmann Patentanwälte PartG mbB
(86) International application number: PCT/JP2024/019302
(87) International publication number: WO 2025/057493

(57) **Abstract**

There is provided a process development support system capable of creating a small scale experimental condition based on a specific culture condition on a large scale even when experimental data on a large scale is insufficient. A process development support system (S) according to the invention includes: a calculation unit (41) configured to perform a simulation in a case of culturing a culture target in culture equipment of a second scale under a second culture condition, based on experimental data including information on a change amount of a plurality of parameters obtained by culturing the culture target in first scale culture equipment under a first culture condition and based on second scale equipment information including at least shape information of the second scale culture equipment larger than the first scale culture equipment, and output, based on a result of the simulation, condition range information indicating a relationship between the second culture condition and the plurality of parameters; and a planning unit (42) configured to output a culture condition of a culture experiment in the first scale culture equipment based on the experimental data and the condition range information.

## Description

### Technical Field

The present invention relates to a process development support system and a process development support method.

### Background Art

From the context of SDGs, climate change issues, realization of carbon neutrality, and the like, it is required to achieve both continuous economic growth and solution of social problems. In the field of manufacturing, there is a growing need for alternatives to petroleum products, and advances in biotechnology have led to a rise in attention to biomanufacturing (production of substances using microorganisms) that makes maximum use of biological functions, such as through smart cell development.

The starting point for the industrialization of biomanufacturing is the development of microorganisms that make the most of the biological functions of the organisms themselves, such as smart cell development. Thereafter, process development for determining culture conditions and scale-up technology are required for industrialization of cultivation in culture tanks exceeding several thousand liters.

In particular, in process development, after a microorganism capable of producing a substance is selected, small scale experiments at the level of several millimeter liters to several liters are carried out to determine culture conditions for production. Here, it is necessary to determine the requirements for preparing a culture medium, oxygen supply, and culture environment for production. For this reason, an optimal culture condition is derived by combining multiple conditions and ranges of controllable parameter items.

However, since the number of combinations is enormous, in view of time and cost, in general, the culture conditions are searched for by a combination of conditions within a very limited range depending on experience values. Therefore, when there are a large number of combinations, an efficient search for optimal culture conditions may be carried out using a computer. As a related art, there is a technique disclosed in PTL 1.

PTL 1 discloses "a cell culture process search method including a process condition generation step of generating a plurality of process conditions for culturing cells, a culture result prediction step of acquiring a culture prediction result of cells for each of the plurality of process conditions generated in the process condition generation step, and an optimal process condition acquisition step of finding an optimal process condition based on the culture prediction results obtained in the culture result prediction step".

In addition, the productivity of a culture condition obtained in the small scale experiment is confirmed in a large scale culture tank having a capacity of several hundred liters or several thousand liters or more. In particular, in the scale-up, it is common to maintain similar geometric shapes and carry out the culture under uniform conditions that keep operating factors constant through chemical engineering calculations for input power and the like. However, this examination alone may not be enough to fully utilize the performance of the microorganisms, resulting in reduced productivity. Therefore, in reality, engineers respond by intuitively tuning and fitting the supply of oxygen and control of the stirring speed based on their past experience. However, this takes time and effort, and there are often cases where the cause cannot be identified. Furthermore, since productivity decreases without identifying the cause, there is a need to go back to and verify the small scale experiment again, which not only increases the number of large scale experiments but also takes a considerable amount of time.

Further, in the culture conditions obtained by the combination of conditions within a limited range of the small scale, even when the same conditions are used, environmental conditions vary due to a difference in the conditions of the culture tank or an increase in the scale. Therefore, a condition of a large scale is not reflected, and the productivity may be impaired. Therefore, a technique for improving the efficiency of a cycle for feeding back results of a small scale and a large scale has been developed. As a related art, there is a technique disclosed in PTL 2.

PTL 2 discloses "a computer implementation method for designing a first scale experiment for an organism so as to generate first scale performance data to be used when predicting performance of the organism in a larger second scale, the method including: a step a of determining a first scale screening condition based at least in part on contribution of a second scale condition to a performance parameter of a first strain of an organism in the second scale, the first scale screening condition including one or more surrogates for the second scale condition that is not reproducible in the first scale; a step of b of determining a first scale screening parameter based at least in part on computer modeling of metabolism of the organism at the second scale; and a step of c of designing an experiment to experimentally screen a second strain of the organism under the first scale screening condition based at least in part on the first scale screening parameter".

### Citation List

### Patent Literature

PTL 1: WO2021/166824
PTL 2: JP2022-531464A

### Summary of Invention

### Technical Problem

With only data about the culture conditions determined in the small scale experiment at the level of several millimeter liters to several liters, an uncertain condition that cannot be reproduced or an unassumed constraint condition exists in a large scale culture tank of several hundred liters or several thousand liters or more that is scaled up thereafter. When the condition is not taken into consideration, productivity cannot be obtained on a large scale, and a lot of time and man-hours are required to examine the experiment on a small scale again. Therefore, in order to efficiently perform process development for scale-up, it is desired to conduct a culture experiment while taking into consideration conditions specific to a large scale different from a small scale. However, in order to conduct experiments for attempting various culture conditions on a large scale, it takes a lot of time and cost for process development, and dedicated equipment is required. Therefore, it is difficult to sufficiently secure experimental data on a large scale.

Therefore, in order to improve the efficiency of the examination of scale-up, there is a demand for a system capable of creating a small scale experimental condition based on a specific culture condition on a large scale, such as a system capable of comparing a difference in behavior of the same parameter between a small scale and a large scale even when experimental data on a large scale is insufficient. In PTL 1 and PTL 2, the above-described issue is not considered.

The invention has been made in view of the above circumstances. An object of the invention is to provide a process development support system and a process development support method capable of creating a small scale experimental condition based on a specific culture condition on a large scale even when experimental data on a large scale is insufficient.

### Solution to Problem

A process development support system according to the invention for solving the above problem is configured including: a storage unit configured to store experimental data including information on a change amount of a plurality of parameters indicating a state in first scale culture equipment in a case where a culture target is subjected to a culture experiment under a first culture condition in the first scale culture equipment, and second scale equipment information that is information on an equipment condition including at least shape information of second scale culture equipment larger than the first scale culture equipment; a calculation unit configured to perform, based on the experimental data and the second scale equipment information, a simulation related to a change amount of parameters same as the plurality of parameters in a case where the culture target is cultured under a second culture condition in the second scale culture equipment, and output, based on a result of the simulation, condition range information that is information indicating a relationship between the second culture condition and the plurality of parameters; and a planning unit configured to output a culture condition of the culture experiment in the first scale culture equipment based on the experimental data and the condition range information.

### Advantageous Effects of Invention

The invention can provide a process development support system and a process development support method capable of creating a small scale experimental condition based on a specific culture condition on a large scale even when experimental data on a large scale is insufficient. Problems, configurations, and effects other than those described above will become apparent from the following description of embodiments.

### Brief Description of Drawings

[FIG. 1] FIG. 1 is a configuration diagram of a process development support system according to an embodiment.
[FIG. 2A] FIG. 2A is an illustrative diagram illustrating an example of a principle of a small scale experimental condition search program.
[FIG. 2B] FIG. 2B is an illustrative diagram illustrating another example of the principle of the small scale experimental condition search program.
[FIG. 2C] FIG. 2C is an illustrative diagram illustrating another example of the principle of the small scale experimental condition search program.
[FIG. 2D] FIG. 2D is an illustrative diagram illustrating another example of the principle of the small scale experimental condition search program.
[FIG. 2E] FIG. 2E is an illustrative diagram illustrating recommendation of a more preferable first scale experimental range.
[FIG. 3] FIG. 3 is an illustrative diagram illustrating a solution concept in the process development support system according to the embodiment.
[FIG. 4] FIG. 4 is an illustrative diagram illustrating an example in which a calculation unit outputs information on a device that meets a first scale recommended experimental condition.
[FIG. 5] FIG. 5 is an illustrative diagram illustrating an example in which the calculation unit outputs information on a device that meets a second scale recommended experimental condition.
[FIG. 6] FIG. 6 is a flowchart illustrating an example of a culture equipment matching program.
[FIG. 7] FIG. 7 is a flowchart illustrating content of a process development support method according to an embodiment.
[FIG. 8] FIG. 8 is a flowchart illustrating a specific example of the process development support method according to the embodiment.

### Description of Embodiments

Hereinafter, embodiments of the invention will be described in detail with reference to the accompanying drawings. In the following description, the same components are denoted by the same terms and reference signs, and a redundant description may be omitted.

In the following embodiment, an example in which a process development support system S according to an embodiment is applied to process development for scaling up a culture process performed in a small scale (referred to as a first scale) and culturing in a large scale (referred to as a second scale) will be mainly described, and the embodiment of the process development support system S according to the embodiment is not limited to this example. For example, the process development support system S according to the embodiment is applicable to a case of culturing microorganisms, cells, fungi, and the like, and a culture target is not limited to a specific organism. A capacity of the first scale is at least smaller than a capacity of the second scale that is scaled up. The capacity is not particularly limited, and the first scale may be a scale at a laboratory level for the purpose of acquiring culture conditions in an experiment. The first scale is preferably at a level of several mL to several tens of L since the handling is easy and a plurality of experiments are easy to be conducted. Further, equipment specifications of the first scale are preferably close to equipment specifications of the second scale, but are not necessarily the same, and culture tanks and equipment that are generally used may be adopted. On the other hand, the second scale may be a bench scale, a pilot scale, or a production scale. The second scale may be, for example, a scale of about several tens of liters to several tens of thousands of liters. In the embodiment, the first scale and the second scale do not indicate a capacity of a certain fixed value, but indicate a capacity in a range having a predetermined width.

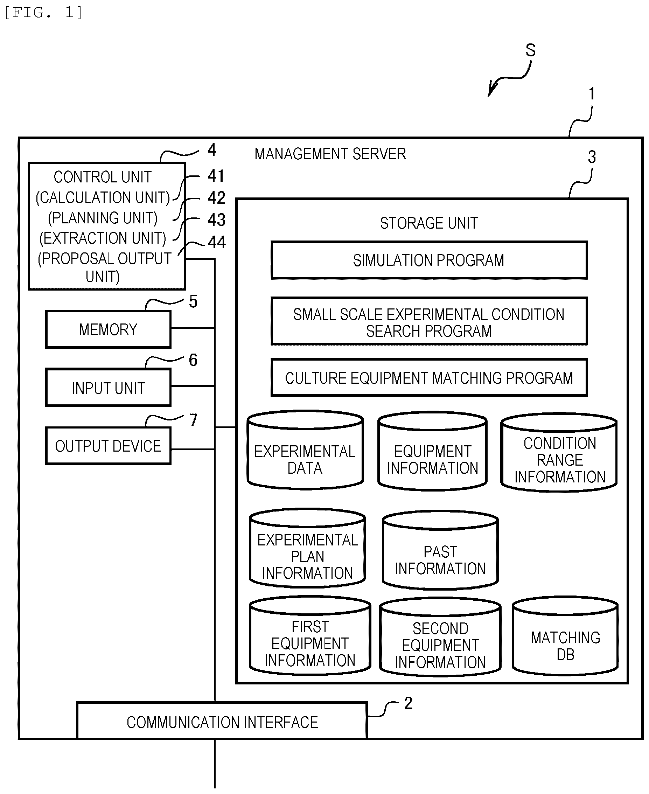

FIG. 1 is a configuration diagram of the process development support system S according to the embodiment. The process development support system S according to the embodiment is embodied as a management server 1 as an example. The management server 1 is a computer including a communication interface 2, a storage unit 3, a control unit 4, a memory 5, an input unit 6, and an output device 7. The communication interface 2 communicates with servers other than the management server 1, user terminals, and culture equipment.

The storage unit 3 stores various types of information such as experimental data, equipment information, condition range information, experimental plan information, past information, first equipment information, second equipment information, and a matching database (DB). The storage unit 3 stores various programs such as a simulation program, a small scale experimental condition search program, and a culture equipment matching program, which will be described later. The control unit 4 is, for example, a central processing unit (CPU), and implements various functions by reading and executing the programs stored in the storage unit 3. In the embodiment, examples of the various functions include a calculation unit 41 and a planning unit 42 described later. In the embodiment, examples of the various functions include an extraction unit 43 and a proposal output unit 44 described later.

The programs and the various functions do not necessarily have a one-to-one correspondence. Processing of reading and executing one program may correspond to two or more different functions, or processing of reading and executing two or more programs may correspond to one function. Therefore, in the embodiment, the calculation unit 41, the planning unit 42, the extraction unit 43, the proposal output unit 44, and the like are shown as configurations corresponding to the functions implemented by the control unit 4, and each function is implemented by reading and executing one or more programs among the simulation program, the small scale experimental condition search program, and the culture equipment matching program. In addition, the classification of functions or programs in the embodiment is an example, and is not limited to this example.

The memory 5 is a random access memory, a read-only memory, or the like. The input unit 6 is a keyboard, a pointing device, or the like. The output device 7 is a display, a printer, or the like. The input unit 6 and the output device 7 are direct input and output interfaces for the management server 1.

Specific roles of the storage unit 3, the calculation unit 41, and the planning unit 42 in the embodiment are as follows.

The storage unit 3 stores experimental data as well as second scale equipment information. The experimental data is experimental data obtained by culturing a culture target in first scale culture equipment. The experimental data includes information on a change amount of a plurality of parameters indicating a state in the first scale culture equipment when the culture target is subjected to a culture experiment under a first culture condition in the first scale culture equipment. The first culture condition refers to a culture condition under which the culture target is cultured in the first scale culture equipment.

The experimental data may include information on an organism that is the culture target, information on a culture environment including control information of the culture equipment, and information on a product produced by the organism that is the culture target. Representative examples of items included in the information on the organism that is the culture target include a type of the organism, characteristics thereof, a passage number, and a seeding density. These items can be used as culture conditions under which the culture target is cultured in the first scale culture equipment.

Representative examples of items included in the information on the culture environment including the control information of the culture equipment include an aeration amount, a stirring rotation speed, a pressure, information on air bubbles, a temperature, a pH, a dissolved oxygen (DO) concentration, a dissolved carbon dioxide (DCO₂) concentration, a culture medium concentration, additive information, and a liquid amount. Set values of these items can be used as culture conditions under which the culture target is cultured in the first scale culture equipment. In addition, regarding these items, information on actual values acquired by the experiment can be used as the information on the change amount of the plurality of parameters indicating the state in the first scale culture equipment.

Representative examples of items included in the information on the product produced by the organism that is the culture target include results such as the cultured product and various component amounts of metabolism. These items can also be used as the information on the change amount of the plurality of parameters indicating the state in the first scale culture equipment. However, the parameter items used as the experimental data are not limited thereto.

The equipment information is information on equipment conditions including at least shape information of the culture equipment. The equipment information includes at least the second scale equipment information. The second scale equipment information is information on equipment conditions including at least shape information of the second scale culture equipment larger than the first scale culture equipment.

The culture equipment includes, in addition to a culture tank, equipment that supplies a culture medium, an aerator that supplies air and oxygen, a stirrer that performs stirring inside the tank, and various control devices that control a pH and an oxygen concentration. Examples of the equipment conditions including at least shape information of the culture equipment include a capacity and an aspect ratio of the culture tank, a ratio of a tank diameter to a blade diameter, a shape of a stirring blade, and other auxiliary equipment information. The equipment information may be information of a drawing comprehensively including the above information.

The matching DB stores information on candidates for the culture tank or a culture device corresponding to the second scale culture equipment. Information such as specifications related to the candidates for the culture tank or the culture device, a name, a manufacturer, a provider, a base, a location, a cost for use, a usage history, a usage status, peripheral equipment, and the like is stored in the matching DB. Information on the specifications includes a chassis to be cultured, a product, a tank capacity, device specifications, and the like for the culture tank or the culture device.

The past information is information for specifying the type and range of parameters that affect a culture result to the extent that a predetermined criterion is satisfied. The past information is, for example, information on past performance data of a culture process in the first scale or the second scale. Based on the performance data, it is possible to extract the type and range of the parameters that affect the culture result to the extent that the predetermined criterion is satisfied. The extraction can be performed by analyzing and evaluating a relationship between each parameter and an index of the culture result by using a method such as machine learning or a statistical method. Further, an existing clustering analysis technique may be used. Further, the past information may be literature information regarding the culture process in addition to the performance data of the culture process. The past information may store information on important parameters, which are parameters that are already known and may play an important role in the purpose of the culture or in a stage of culture process development, or information that indicates a specific range of such parameters. The past information may be information acquired from an external server using the communication interface 2. Other information and programs will be described later.

The information stored in the storage unit 3 is not limited to the various types of information and data described above. The storage unit 3 can accumulate, for example, information on the first scale culture equipment, and culture experimental data in the second scale culture equipment obtained after operating the process development support system S according to the embodiment. In the embodiment, necessary data may be held in the storage unit 3 of the management server 1, or may be distributed and stored in a plurality of servers connected via a network.

Based on the first scale experimental data and the second scale equipment information, the calculation unit 41 performs a simulation related to a change amount of the parameters same as the plurality of parameters in a case of culturing the culture target under a second culture condition in the second scale culture equipment. The calculation unit 41 outputs condition range information, which is information indicating a relationship between the second culture condition and the plurality of parameters, based on a result of the simulation. The second culture condition refers to a culture condition under which the culture target is cultured in the second scale culture equipment.

The condition range information is information created by the simulation program, that is, the calculation unit 41. The condition range information is information indicating a relationship between the culture condition (second culture condition) and the various parameters that may affect the culture in the second scale culture equipment. The condition range information may be information indicating behaviors of the various parameters that may affect the culture when the second culture condition is changed in the second scale culture equipment. The condition range information may be information indicating a range of values that the various parameters can take in the second scale culture equipment. Here, the second culture condition may be any experimental condition that can be simulated in the simulation. Examples of the culture conditions include initial conditions such as viscosity and various concentrations, and operation conditions such as set values of temperature, set values of pH, rotation speed, and aeration. Examples of the various parameters that may affect the culture include a flow velocity distribution, a shear stress distribution, a temperature change amount, a pH change amount, an oxygen supply rate, a DO concentration, a DCO₂ concentration, heat transfer, substance transfer, and gas dispersion in the culture tank.

The condition range information may include information on values that the various parameters can take for each culture condition. With this information, it is possible to calculate information on a degree of influence of the culture condition on changes of the various parameters. The degree of influence of the culture condition on changes of the various parameters may be specified as a number of evaluation levels. In this case, the degree of influence is specified to be larger as the influence on the various parameters when changing the culture condition is greater. As an example, a magnitude of the influence of the culture condition on changes of the various parameters is quantitatively calculated based on information on a ratio of change of the various parameters when the culture condition is changed by a predetermined ratio. The degree of influence of the culture condition on changes of the various parameters may be a value calculated for each type of the various parameters.

The planning unit 42 outputs a culture condition (experimental plan information) of a culture experiment in the first scale culture equipment based on the experimental data and the condition range information. The culture condition output here can be a first culture condition of the culture experiment in the first scale culture equipment to be performed next time.

The experimental plan information is information created by the small scale experimental condition search program, that is, the planning unit 42. The experimental plan information is information on an experimental plan in the first scale culture equipment that is necessary for process development for culturing the culture target in the second scale culture equipment. With the experimental plan information, the creation of an experimental plan in the first scale culture equipment can be supported. The experimental plan information may be in any form according to a culture system, a process system, and a management mode. The experimental plan information may be, for example, information indicating a range of values to be set for parameters that can be set in the first scale culture equipment. The experimental plan information may include information on specific culture conditions of the culture experiment, an experimental procedure, and an experimental method.

The planning unit 42 can output information for recommending culture equipment to be used in the first scale. In this case, information on the experimental procedure and the experimental method related to the culture equipment to be used in the first scale may also be output. The planning unit 42 may output information on candidates for the culture equipment to be used as the second scale culture equipment after the scale-up.

The simulation program is a program for simulating behaviors of the various parameters (referred to as secondary parameters) when performing culture in the second scale culture equipment, based on predetermined culture conditions and information (referred to as primary parameters) on the second scale culture equipment. The simulation program can be executed by the calculation unit 41. The simulation program can use, for example, a commonly used fluid analysis (CFD) simulation.

In the simulation assuming a parameter change amount of the second culture condition from the first scale experimental data and the second scale equipment information, for example, fluid analysis can be used in addition to chemical engineering calculation. Examples of the primary parameters include an operation condition such as a stirring rotation speed, an aeration amount, and a volume, and an equipment setting condition such as a shape of the stirring blade and an aspect ratio in the tank. Examples of the secondary parameters calculated based on these parameters include a shear stress, a gas and liquid amount such as a DO concentration and a DCO₂ concentration, a velocity, energy, a mixing time, a product and substrate concentration distribution, a flow velocity distribution, a Kolmogorov scale, foaming, and a carbon dioxide partial pressure (pCO₂), and a pH distribution, and a simulation result desirably indicates a distribution state thereof. The behaviors of the secondary parameters described above can be simulated by using a commonly used fluid analysis technique based on the information of the primary parameters described above. From these, it is possible to show relationships between the culture conditions and the parameters, and to extract, acquire, and output the condition range information of each parameter. The simulation by the simulation program shows that the distribution of the DO concentration in the second scale culture equipment is 1 mg/L to 5 mg/L, for example.

The small scale experimental condition search program is a program for creating, based on a result of the simulation by the simulation program, the experimental plan information in the first scale culture equipment that is necessary for process development for culturing the culture target in the second scale culture equipment. The small scale experimental condition search program can be executed by the planning unit 42. An example of a principle of the small scale experimental condition search program will be described later.

Based on the condition range information, the small scale experimental condition search program analyzes a difference between a behavior in the first scale culture equipment and a behavior in the second scale culture equipment for a plurality of parameters that may affect the culture result. The small scale experimental condition search program extracts an important parameter to be focused on when scaling up a target process based on an analysis result. The small scale experimental condition search program may create recommendation information regarding an experimental plan by using information on the extracted important parameter. The important parameter is a parameter that may play an important role in the purpose of the culture or in the stage of culture process development, or is a specific range of the parameter.

The small scale experimental condition search program outputs a condition if the parameter can be directly set in the first scale culture equipment. The small scale experimental condition search program can output a culture condition (a new first culture condition) by an operation of converting into a parameter to be examined in the first scale, for example, chemical engineering calculation. The small scale experimental condition search program particularly outputs a range in which set values of items of a plurality of operation conditions are changed.

From the experimental data of the first scale and the acquired condition range information, the small scale experimental condition search program can output information on important parameters to be considered in the second scale culture equipment based on items that are not significantly reflected in the experimental data, items that are not examined, and the condition range information. The small scale experimental condition search program can use clustering analysis or the like when extracting important parameters or the like. For example, the clustering analysis is preferably performed for new experimental data obtained by performing a culture experiment under newly output culture conditions (first culture conditions) of the culture experiment in the first scale culture equipment. The clustering analysis is preferably repeated every time new experimental data is obtained. When the clustering analysis is performed as described above, accuracy of the experimental plan information in the first scale culture equipment can be improved.

The small scale experimental condition search program can output culture conditions of the culture experiment to be examined in the first scale culture equipment, set a condition range, and propose and recommend conditions of the culture equipment for acquiring the culture conditions. For example, when there is a substrate concentration distribution, it is possible to propose a device capable of reproducing the concentration distribution. Furthermore, the shape of the stirring blade or the like is also proposed.

In consideration of the important parameters and the condition range information, the small scale experimental condition search program can further propose and recommend conditions of the second scale culture equipment required to minimize items with large distributions and parameters with high influence. The small scale experimental condition search program can propose and recommend, for example, the shape of the culture tank, the stirring blade used in the culture tank, a stirring condition, and an air diffuser condition for a bubble diameter. The small scale experimental condition search program can further propose information on culture equipment to be recommended (first scale culture equipment), based on culture equipment information in the storage unit 3 or the like. In the case of recommendation of the equipment, an installation location, an operation status, a usage history, and the like may be indicated in addition to equipment information and specifications.

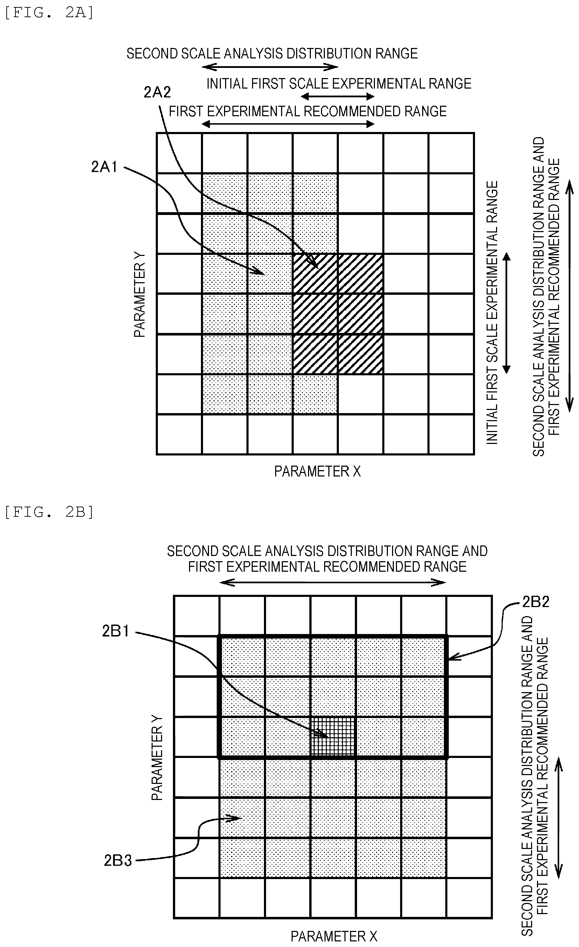

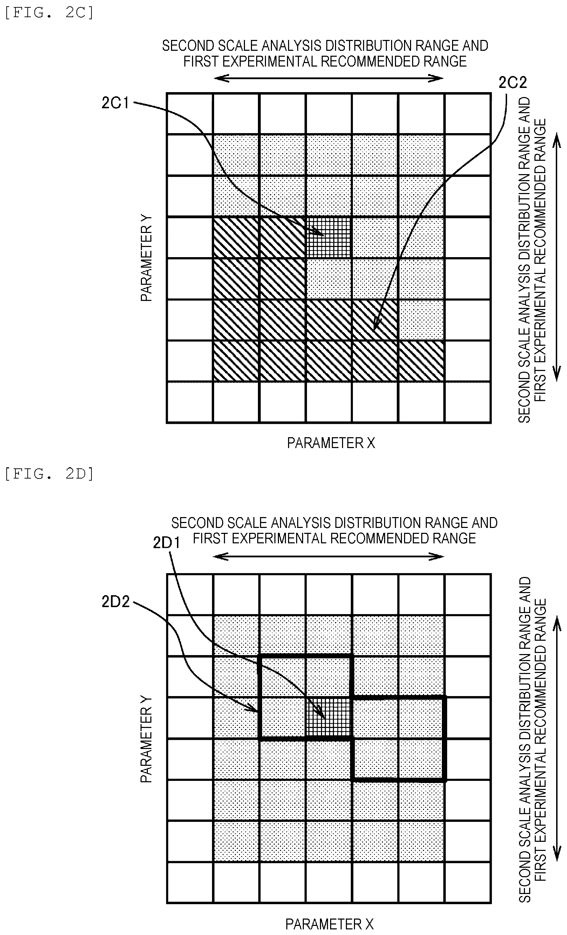

Next, an example of the principle of a small scale experimental condition search program will be described with reference to FIGS. 2A to 2D. FIGS. 2A to 2D are illustrative diagrams illustrating an example of the principle of the small scale experimental condition search program. The small scale experimental condition search program extracts an important parameter as described above. The important parameter is a parameter that may play an important role in the purpose of culture or in the stage of culture process development, or a specific range of the parameter. There are several extraction methods for the specification, and some content thereof will be described as an example of the principle of the small scale experimental condition search program, and the invention is not limited to the following method.

[1] As illustrated in an extraction diagram 2A by scale difference, when an analysis distribution range (second scale analysis distribution range 2A1) in the second scale culture equipment for parameters X and Y of certain analyzed parameter candidates has a range not included in an initial experimental condition range (initial first scale experimental range 2A2) of the first scale culture equipment, the planning unit 42 can extract the parameter in which the range is present or the parameter of the range as an important parameter. Alternatively, when a ratio of the range not included in the initial experimental condition range of the first scale culture equipment to the analysis distribution range in the second scale culture equipment is larger than a predetermined threshold, the corresponding parameter may be extracted as the important parameter. Accordingly, a parameter in which the second scale analysis distribution range 2A1 is significantly deviated from the initial first scale experimental range 2A2 can be recognized as an important parameter in culture process development. The predetermined threshold may be freely selected for each parameter. The initial first scale experimental range 2A2 and the second scale analysis distribution range 2A1 are also first experiment recommended ranges.

A degree of influence of each parameter in culture process development may be evaluated. Here, the degree of influence in the culture process development may be calculated based on information on the ratio of the range not included in the initial first scale experimental range 2A2 to the second scale analysis distribution range 2A1. As an example, the degree of influence may be a relative value of a change width of the range, of the second scale analysis distribution range 2A1, not included in the initial first scale experimental range 2A2 in a case where a change width of the initial first scale experimental range 2A2 is defined as 1. In this case, the greater the relative value, the greater the degree of influence in the culture process development. Accordingly, it is possible to recognize a parameter, in which the second scale analysis distribution range 2A1 is greatly deviated from the initial first scale experimental range 2A2, as a parameter having a large degree of influence in the culture process development.

[2] As illustrated in an extraction diagram 2B from the viewpoint of hardware, as a result of analyzing the second scale for the parameters X and Y of certain analyzed parameter candidates, in a case where, in an experimental condition (first scale Input experimental condition 2B1) input for the first scale culture equipment, a condition (range 2B2 that is difficult to control in the second scale) by which the first scale Input experimental condition 2B1 cannot be realized in the second scale culture equipment is found, the planning unit 42 can extract the parameter in which the range 2B2 is present or the parameter of the range 2B2 as an important parameter. Alternatively, when a ratio of the range that cannot be realized in the second scale culture equipment to the range of the first scale Input experimental condition 2B1 is larger than a predetermined threshold, the parameter may be extracted as an important parameter. Accordingly, a parameter having a large overlap between the initial first scale experimental range and the range that cannot be used in the second scale can be recognized as an important parameter in the culture process development. In this case, the planning unit 42 can designate a first experiment recommended range (second scale analysis distribution range 2B3) excluding the range 2B2 that is difficult to control in the second scale.

The planning unit 42 may evaluate a degree of influence of each parameter in the culture process development. Here, the degree of influence in the culture process development may be calculated based on information on the ratio of the range that cannot be realized in the second scale culture equipment to the range of the first scale Input experimental condition 2B1. In this case, the degree of influence in the culture process development is evaluated to be larger as the ratio is larger. Accordingly, a parameter having a large overlap between the initial first scale experimental range and the range that cannot be used in the second scale can be recognized as a parameter having a large degree of influence in the culture process development.

In the embodiment, as an additional configuration for improving accuracy, the following "extraction as past information" and "extraction from additional examination" may be added.

[3] As illustrated in an extraction diagram 2C as past information, the planning unit 42 can extract a parameter and a parameter range considered to be important in the past with respect to a first scale Input experimental condition 2C1 for the parameters X and Y of parameter candidates based on past information stored in the storage unit 3. The parameter can be extracted from information that may be specifically affected in an input culture target. That is, the parameter can be extracted as a range affected by performance or a range 2C2 determined to be affected by past information in an experiment on the first scale. With respect to the past information, it is also possible to check necessary information from a generally used literature or the like. It is also possible to freely input information on an important parameter, which is found in advance, to the storage unit 3 as past information. In particular, when a condition of a parameter affected is close to an input experimental condition in the first scale culture equipment, it is desirable to extract the parameter from the past information such as the information on the important parameter. It is desirable that a degree of closeness can be freely selected. As described above, by extracting parameters and parameter ranges considered to be important in the past and considering the parameters and the parameter ranges as information on an order or priority when creating an experimental plan in the first scale, it is possible to contribute to the improvement in efficiency of the experiment in the first scale.

[4] As illustrated in an extraction diagram 2D from the additional examination, the planning unit 42 can recommend an additional experiment in advance as necessary in a case where the number of parameters in an input first scale Input experimental condition 2D1 or information thereof is insufficient as compared with past examination based on the past information stored in the storage unit 3. It is desirable that the condition can be freely selected. In addition, it is also possible to perform an experiment for measuring the degree of influence or an experiment for specifying an influence factor, as necessary from the information such as the past information or a scale difference, and it is also possible to recommend the experiments. As these experiments, it is conceivable to perform an experiment (experiment of first scale initial experimental range 2D2) in which an experimental condition range (the second scale analysis distribution range and the first experiment recommended range) is widened. Thus, the important parameter can be extracted again. In a case where it is difficult to specify a parameter that may exert influence, for example, it is also possible to analyze a culture component from an experiment having the influence or having a possibility of influence, derive a difference by using a method such as machine learning or a statistical method, and extract the difference as an important parameter. Furthermore, it can be estimated and derived from information such as a metabolic pathway of a target culture. The important parameter can be extracted by adding these pieces of information to the storage unit 3 and the planning unit 42.

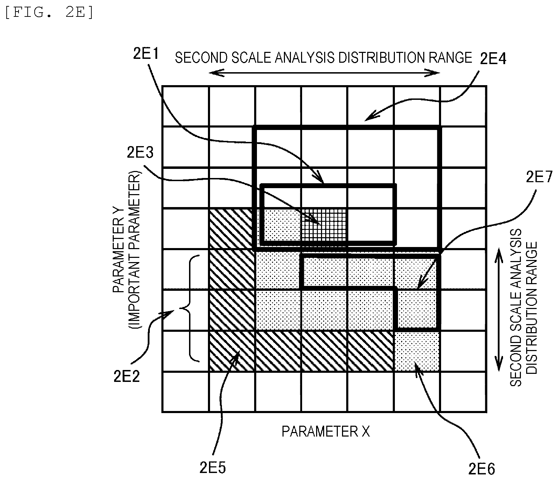

In the embodiment, [1] to [4] can be appropriately combined and applied. Accordingly, it is possible to more appropriately grasp which range should be experimented in the first scale culture equipment when considering the second scale culture equipment. That is, a more preferable first scale experimental range can be recommended. Here, FIG. 2E is an illustrative diagram illustrating recommendation of a more preferable first scale experimental range. FIG. 2E illustrates an example in which all of [1] to [4] are applied.

As illustrated in FIG. 2E, when an analysis distribution range (second scale analysis distribution range) in the second scale culture equipment for the parameters X and Y of certain analyzed parameter candidates has a range 2E2 not included in an initial experimental condition range (initial first scale experimental range 2E1) of the first scale culture equipment, the planning unit 42 recognizes that an important parameter is included in the range 2E2 and extracts the important parameter. As a result of analyzing the second scale for the parameters X and Y of certain analyzed parameter candidates, in a case where, in the first scale Input experimental condition 2E3, a condition (range 2E4 that is difficult to control in the second scale) by which a first scale Input experimental condition 2E3 cannot be realized in the second scale culture equipment is found, the planning unit 42 extracts the range 2E4 as an important parameter. Further, for the parameters X and Y of parameter candidates, the planning unit 42 can extract, as an important parameter, a parameter considered to be important in the past in the storage unit 3 with respect to the first scale Input experimental condition 2E3 (for example, a range 2E5 that is greatly affected by performance or a range 2E6 that is determined to be affected by past information in an experiment on the first scale). The planning unit 42 can recommend an additional experiment in advance as necessary in a case where the number of parameters and information thereof in the input first scale Input experimental condition 2E3 are insufficient as compared with past examination. As such an additional experiment, in addition to an increase in the number of samples in the first scale Input experimental condition 2E3, an experiment in an experimental condition range (an experiment in a recommended culture condition range 2E7 that is not exemplified in the ranges 2E1, 2E2, and 2E4 to 2E6) is considered.

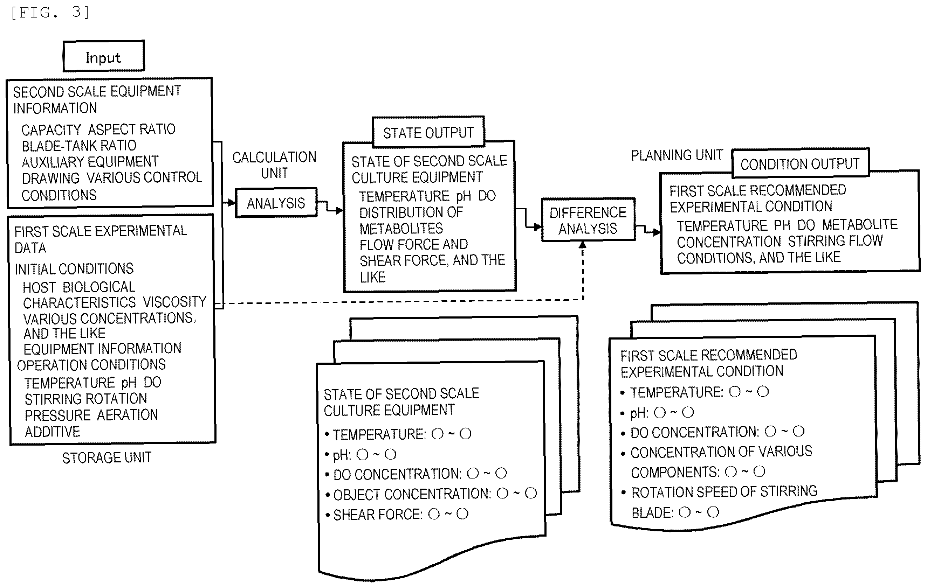

FIG. 3 is an illustrative diagram illustrating a solution concept in the process development support system S according to the embodiment. As illustrated in FIG. 3, the process development support system S according to the embodiment can input first scale experimental data when culturing a culture target in the first scale and scaled-up second scale equipment information, and output, as an output, a first scale recommended experimental condition necessary for process development for culturing the culture target in the second scale culture equipment.

Specifically, the calculation unit 41 performs analysis such as chemical engineering calculation and simulation of a culture environment in the second scale based on the input information. Examples of the input information regarding the first scale experimental data include initial conditions such as a host, biological characteristics, viscosity, various concentrations, and equipment information, and operation conditions such as a temperature, a pH, a DO concentration, stirring rotation, a pressure, aeration, and an additive. Examples of the input information regarding the second scale equipment information include a capacity, an aspect ratio, a blade-tank ratio, auxiliary equipment, drawings, and various control conditions.

Based on these pieces of information, the planning unit 42 analyzes a difference between a behavior in the first scale and a behavior in the second scale for a plurality of parameters that may affect a culture result. In this analysis, for example, a state of the second scale culture equipment such as a temperature, a pH, a DO concentration, a distribution of metabolites, a flow force, and a shear force is output. Examples of the state of the second scale culture equipment include a temperature of ○ ~ ○, a pH of ○ ~ ○, a DO concentration of ○ ~ ○, an object concentration of ○ ~ ○, and a shear force of ○ ~ ○.

Based on an analysis result, the planning unit 42 performs difference analysis on an important parameter to be focused on when scaling up a target process, and extracts the important parameter, for example, as in [1] to [4] described above. The planning unit 42 creates recommendation information (newly output first culture condition) regarding the first scale recommended experimental condition by using information on the extracted important parameters, and outputs the recommendation information to the output device 7 as output information. Examples of the first scale recommended experimental condition include a temperature of ○ ~ ○, a pH of ○ ~ ○, a DO concentration of ○ ~ ○, a concentration of various components of ○ ~ ○, and a rotation speed of a stirring blade of ○ ~ ○. At this time, as a method of deriving the first culture condition from the information on the important parameter, for example, conversion can be performed based on a chemical engineering theoretical formula (theoretical model) indicating a relationship between various parameters and variables of the culture condition. As a different method, the first culture condition may be derived from parameter information based on information regarding a correspondence relation between the culture condition and the parameter included in condition range information. As an example, as a method of deriving the first culture condition from the state of the second scale culture equipment, from the viewpoint of chemical engineering, the shear force is determined by specifications of the stirrer including the shape and the rotation speed of the stirring blade. When a culture state of the shear force of the second scale culture equipment has a range, for example, based on a value that the shear force can take, the rotation speed of the stirrer in the first scale culture equipment can be found from the shape of the stirring blade of the stirrer in the first scale culture equipment. This rotation speed condition can be output as the first scale recommended experimental condition.

When outputting the recommendation information regarding the first scale recommended experimental condition, the planning unit 42 can output information on a priority in the first culture condition or information on importance of the first culture condition based on information regarding the magnitude of influence of various parameters in the case of culturing the culture target in the second scale culture equipment that is output by the planning unit 42. As an example, an experiment using a culture condition corresponding to a parameter evaluated to have a high degree of influence may be determined to have a high priority, and information on the priority of the culture condition may be output together with information on the culture condition. As an example, a culture condition corresponding to a parameter evaluated to have a high degree of influence may be determined to have a high degree of influence, and information on the degree of influence of the culture condition may be output together with the information on the culture condition.

As described above, the process development support system S according to the embodiment can perform an experiment in the first scale culture equipment in consideration of a specific culture condition in the second scale culture equipment. Accordingly, in the process development support system S according to the embodiment, even if there is no experimental data information in the second scale culture equipment, an uncertain condition that cannot be reproduced or an unassumed constraint condition is almost eliminated, and a decrease in productivity in the second scale due to the culture equipment can be prevented.

### (Preferred Aspects)

Preferred aspects of the process development support system S according to the embodiment described above will be described below.

### (Aspect 1)

The first culture condition preferably includes information on set values of items of a plurality of operation conditions (culture conditions) for controlling the first scale culture equipment. The planning unit 42 preferably outputs, based on experimental data and condition range information, a range in which the set values in a culture experiment are changed. In other words, it is desirable that an input value and an output value of the process development support system S are the set values of the items of the operation conditions (culture conditions). Here, a method of outputting the range in which the set value of the culture condition in the culture experiment is changed can be performed by the method of extracting the important parameter and the method of deriving the first culture condition from the information on the important parameter described above. In this way, since a possible range of the primary parameter (explanatory variable) of the culture experiment in the first scale culture equipment can be determined, an experimental plan of the first scale culture equipment can be more appropriately created by the calculation unit 41 and the planning unit 42.

### (Aspect 2)

The planning unit 42 preferably outputs information on the magnitude of the influence in the case of culturing the culture target in the second scale culture equipment, based on information on a plurality of parameters included in the experimental data and information on the plurality of parameters included in the condition range information. The information on the magnitude of the influence in the case of culturing the culture target in the second scale culture equipment may be information on the degree of influence of the various parameters described above. In this way, since it is possible to specify a secondary parameter (that is the same as the primary parameter in this aspect) that is important when performing culture in the second scale culture equipment, it can be expected that the culture can be more suitably performed when actually performing the culture in the second scale culture equipment.

### (Aspect 3)

The storage unit 3 preferably stores first equipment information regarding a plurality of candidates for the first scale culture equipment. For example, similarly to items included in the equipment information, the first equipment information may store information on equipment conditions including at least shape information of the first scale culture equipment. In addition to specifications of the first scale culture equipment, the first equipment information may also store information such as a name, a manufacturer, a provider, a base, a location, a cost for use, a usage history, a usage status, and peripheral equipment. The first equipment information may be information in a plurality of types of standard formats that is related to device specifications. In addition, the planning unit 42 preferably outputs information on the culture equipment to be used for the culture experiment in the first equipment information. The information on the culture equipment to be used for the culture experiment in the first equipment information may be information on a device condition satisfying a first scale recommended experimental condition.

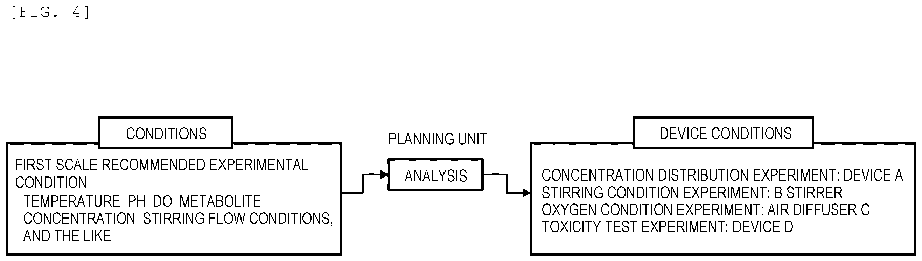

FIG. 4 is an illustrative diagram illustrating an example in which the planning unit 42 outputs information on a device that meets a first scale recommended experimental condition. As illustrated in FIG. 4, when a condition (first scale recommended experimental condition) is input from the input unit 6, the planning unit 42 performs analysis based on the first equipment information. The first scale recommended experimental condition may be information output by the planning unit 42. Examples of the first scale recommended experimental condition include a temperature, a pH, a DO concentration, a metabolite concentration, and a stirring flow condition.

Based on an analysis result, the planning unit 42 outputs, as a device condition related to the culture experiment in the first scale culture equipment, information on the device suitable for the first scale recommended experimental condition. Examples of the output device conditions include a device A for a concentration distribution experiment, a B stirrer for a stirring condition experiment, an air diffuser C for an oxygen condition experiment, and a device D for a toxicity test experiment. As described above, the planning unit 42 can output information on a plurality of candidates for a device that meets the first scale recommended experimental condition. When the storage unit 3 and the planning unit 42 perform the processing, the culture equipment to be used for the culture experiment on the first scale can be suitably recommended.

### (Aspect 4)

The storage unit 3 preferably stores second equipment information regarding a plurality of candidates for the second scale culture equipment. For example, similarly to items included in the equipment information, the second equipment information may store information on an equipment condition including at least shape information of the second scale culture equipment. In addition to specifications of the second scale culture equipment, the second equipment information may also store information such as a name, a manufacturer, a provider, a base, a location, a cost for use, a usage history, a usage status, and peripheral equipment. The second equipment information may be information in a plurality of types of standard formats that is related to device specifications. The planning unit 42 preferably outputs information on candidates for the culture equipment to be used as the second scale culture equipment, based on information on a range and a behavior of parameters same as a plurality of parameters, information on an important parameter to be focused on when scaling up a process of the culture target (that is, an important parameter in process development of scale-up from the first scale culture equipment to the second scale culture equipment), and the second equipment information. The information on the candidates for the culture equipment to be used as the second scale culture equipment may be a device condition satisfying the second scale recommended experimental condition.

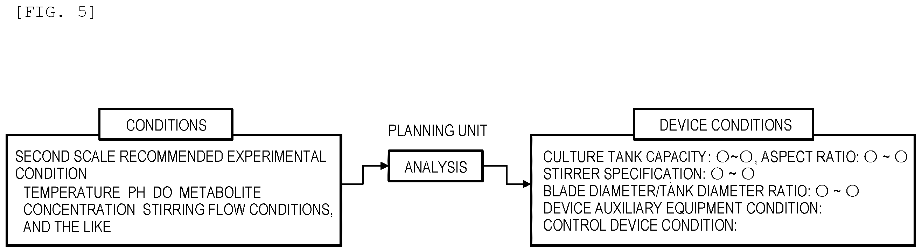

FIG. 5 is an illustrative diagram illustrating an example in which the planning unit 42 outputs information on a device that meets a second scale recommended experimental condition. As illustrated in FIG. 5, when a condition (second scale recommended experimental condition) is input from the input unit 6, the planning unit 42 performs analysis based on the second equipment information. Examples of the second scale recommended experimental condition include a temperature, a pH, a DO concentration, a metabolite concentration, and a stirring flow condition. The planning unit 42 may output the second scale recommended experimental condition necessary for the input together with the recommended experimental condition of the first scale. In this case, the second scale recommended experimental condition may be information on a condition of the second scale culture equipment required to minimize items with large distributions and parameters with high influence based on the important parameters and the condition range information. In this case, as an example, the shape of the culture tank, the stirring blade used in the culture tank, the stirring condition, the air diffuser condition for the bubble diameter, and the like are output.

Based on an analysis result and the second equipment information, the planning unit 42 outputs, as a device condition related to the second scale culture equipment, information on a device that meets the second scale recommended experimental condition. Examples of the output device condition include a culture tank capacity of ○ ~ ○, an aspect ratio of ○ ~ ○, a stirrer specification of ○ ~ ○, a blade diameter/tank diameter ratio of ○ ~ ○, various device auxiliary equipment conditions, and various control device conditions. The planning unit 42 may output, together with the equipment condition satisfying the second scale recommended experimental condition, the information on the candidates for the second scale culture equipment based on information such as specifications, a name, a manufacturer, a provider, a base, a location, a cost for use, a usage history, a usage status, or peripheral equipment of the culture equipment included in the second equipment information. As described above, the planning unit 42 can output information regarding a plurality of candidates for a device that meets the second scale recommended experimental condition. When the storage unit 3 and the planning unit 42 perform the processing, the culture equipment to be used for the culture experiment on the second scale can be suitably recommended.

### (Aspect 5)

The first culture condition is preferably at least one of a viscosity, a concentration of various components, a temperature, a pH, a DO concentration, a stirring rotation speed, an aeration amount, and a pressure. When the first culture condition is selected from these, a culture experiment using the first scale culture equipment can be suitably conducted.

### (Aspect 6)

The equipment condition is preferably at least one of an aspect ratio of the culture tank, an aerator, a stirrer, pH adjustment equipment, a control device that controls a dissolved oxygen concentration, and specifications thereof. When the equipment condition is selected from these, culture using the second scale culture equipment can be suitably performed. The equipment condition can be provided as useful information for matching described later. Examples of the pH adjustment equipment include a device that supplies an acidic agent or an alkaline agent.

### (Aspect 7)

The calculation unit 41 preferably simulates, based on the second culture condition and the second scale equipment information, behaviors of various parameters when culture is performed in the second scale culture equipment. The various parameters are preferably at least one of a shear force and a DO concentration. When the calculation unit 41 performs the above processing and the various parameters are selected from the above, it is possible to suitably perform the culture in the second scale culture equipment.

### (Aspect 8)

In production of useful substances by fermentation using microorganisms or the like, only a person who hold a culture tank can carry out the culture. In addition, the holder often has information on the culture tank. The holder of the culture tank may provide the culture tank through a contract business, but the person who entrusts the business or uses the culture tank does not know the details of the culture tank, and it is difficult to know whether the culture tank is suitable for the culture conditions that the user wants to use. Further, there is a situation in which information on a usable time and an appropriate culture tank is insufficient and a search is performed at random. On the other hand, a rate of operation of the culture tank varies among those who hold the culture tank, and the culture tank is in a non-operating state when the culture tank is not used, which increases the maintenance cost. Therefore, when there are a user who wants to use the second scale culture equipment and a provider (the holder described above) who provides the second scale culture equipment, if the user and the provider can be matched, it is efficient in performing culture in the second scale culture equipment.

Therefore, the process development support system S according to this aspect preferably includes a database configured to store information on candidates for second scale culture equipment and information on specifications and usage of the candidates. The information on the candidates for the second scale culture equipment may be information such as a name, a provider, a base, and a location of each of the candidates for the culture tank or a culture device corresponding to the second scale culture equipment. The information on the specifications of the candidates for the second scale culture equipment may be information on products by the culture equipment, information on a chassis to be cultured, information on device specifications such as a tank capacity, and the like. The information on the usage of the candidates for the second scale culture equipment may be information on a cost, a usage history, a usage status, a reservation status, or peripheral equipment. The information on device specifications may further include an aspect ratio of the culture tank, a ratio of a tank diameter to a blade diameter, a shape of a stirring blade, auxiliary equipment information, and the like. In this aspect, it is possible to more accurately estimate the candidates for the culture equipment suitable for the condition desired by the user by using these pieces of information. Such information in the database may be information acquired from an external server via the communication interface 2. The database described above may be stored in the storage unit 3 as the matching DB.

The process development support system S according to this aspect preferably includes the input unit 6 configured to input a condition of the second scale culture equipment. For example, information on specifications of the culture tank or the culture device corresponding to the second scale culture equipment desired to be used by the user is input. Further, as another example, it is possible to input at least one of the information on the specifications of the culture tank or the culture device corresponding to the second scale culture equipment desired to be used by the user, information on an outline of a product or the like, and a function of the culture tank or the culture device. The function of the culture tank or the culture device may be information on a predetermined function in control during culture in the culture tank or the culture device, or related auxiliary equipment information. In addition to the above, a condition related to information on the usage of the culture equipment may be input.

Further, the process development support system S according to this aspect preferably includes the extraction unit 43 configured to extract, from among the candidates for the second scale culture equipment, one or more candidates for the culture equipment that are related to the condition received by the input unit 6, based on the information received by the input unit 6 and the information stored in the database. As an example, one or more similar culture tanks or culture devices may be extracted from the information stored in the database based on the information on the chassis, the product, and the tank capacity received by the input unit 6. Accordingly, in this aspect, it is possible to accurately extract a candidate for the culture equipment suitable for the condition desired by the user. Further, in order to accurately execute the processing by the extraction unit 43, items in the information on the specifications and usage of the candidates for the second scale culture equipment stored in the database may correspond to items in the condition of the second scale culture equipment received by the input unit 6. Here, the items do not necessarily correspond one-to-one, and an item on one side may correspond to two or more items on the other side. A table defining the correspondence relation may be further stored in the database, and the processing by the extraction unit 43 may be executed based on information in the table.

Further, the process development support system S according to this aspect preferably includes the proposal output unit 44 configured to propose the candidates for the culture equipment extracted by the extraction unit 43 and information on usage of each of the candidates. A plurality of candidates for the culture equipment may be proposed. Further, information on an availability schedule of the culture tank or the culture device that is a candidate for the culture equipment may be proposed as the information on the usage. Here, the information on the availability schedule may be specified from information on a usage history, a usage status, a reservation status, and the like in the database. Together with a candidate for the culture equipment, information on the condition of the second scale culture equipment received by the input unit 6 and on a similarity with the candidate may be output. The information on the similarity may be indicated by a predetermined index. As an example, such an index may be calculated based on the number of items satisfying the condition by comparing the items in the condition of the second scale culture equipment received by the input unit 6 with the corresponding items in the information on the specifications and usage of the candidates for the second scale culture equipment stored in the database. Together with the candidates for the culture equipment, information on a past usage status related to the condition of the second scale culture equipment received by the input unit 6 may be output. With such a configuration, it is possible to support the user to select the second scale culture equipment. The extraction unit 43 and the proposal output unit 44 can be implemented by the control unit 4 executing a program (culture equipment matching program) that achieves these functions.

In this aspect, one or more pieces of usable second scale culture equipment can be extracted and proposed based on the information such as the usage of the culture tank included in the database and the content input by the user. That is, in this aspect, it is possible to provide a matching system that matches a user who wants to use the culture tank with a provider who provides the culture tank or the culture device to the user. Accordingly, in this aspect, it is possible to improve the efficiency of culturing in the second scale culture equipment. That is, this aspect can resolve an issue of providing a matching system that matches a user who wants to use a culture tank with a provider who provides a culture tank or a culture device to the user, and an issue of improving the efficiency of culturing in the second scale culture equipment. In this aspect, an optimal culture tank is proposed so that the user can use the optimal culture tank while knowing the specifications and an available time of the culture tank. Further, it will also reduce vacancies for culture tank holders, leading to development in the bioproduction field.

An example in which the invention according to this aspect is used for a solution for matching culture tanks will be described. As an example, a database stores information on candidates for the second scale culture equipment owned by one or more vendors (owners and providers) capable of providing the second scale culture equipment and information on specifications and usage of each of the candidates, which are acquired via the communication interface 2. Information on second scale culture equipment desired to be used by a cell owner (user) who desires to use the second scale culture tank is received by the input unit 6. Then, after the processing of the extraction unit 43 described above is executed, the proposal output unit 44 may output, on a screen for a manager of the cell owner, the information on the candidates for the culture equipment extracted by the extraction unit 43, information on an owner of each of the candidates, and the information on the usage of each of the candidates.

An example of the process development support system S is configured including: the database (storage unit 3) configured to store the information on candidates for the second scale culture equipment owned by one or more vendors capable of providing the second scale culture equipment and the information on specifications and usage of each of the candidates, which are acquired via the communication interface 2; the input unit 6 configured to receive a condition of the second scale culture equipment related to the cell owner who desires to use the second scale culture equipment; the extraction unit 43 configured to extract, from among the candidates for the second scale culture equipment, one or more candidates for the culture equipment that are related to the condition received by the input unit 6, based on the information received by the input unit 6 and the information stored in the database; and the proposal output unit 44 configured to output, on the screen for the manager of the cell owner, the information on the candidates for the culture equipment extracted by the extraction unit 43, the information on the owner of each of the candidates, and the information on usage of each of the candidates.

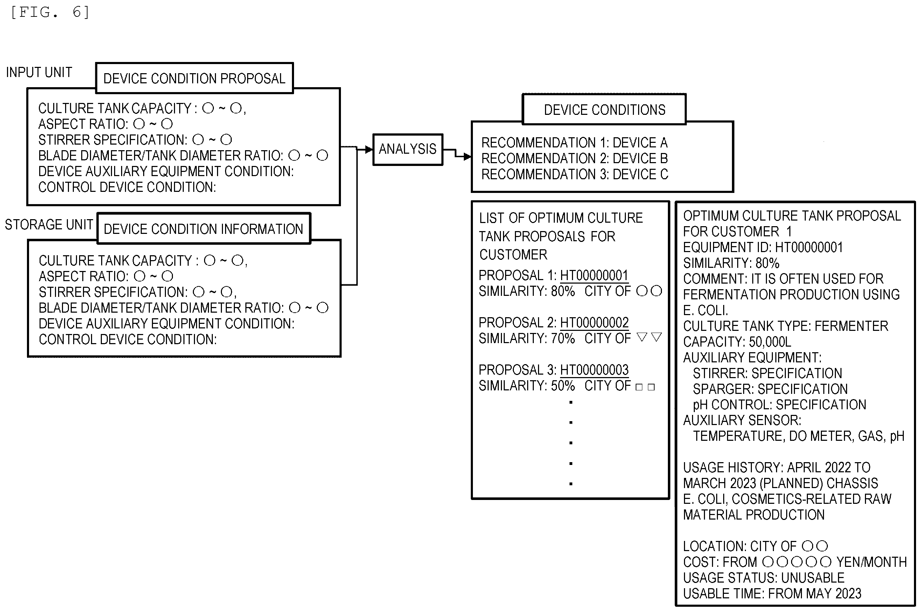

FIG. 6 is a flowchart illustrating an example of culture equipment matching program. As illustrated in FIG. 6, device condition information is stored in the storage unit 3. Examples of the device condition information include specifications of a culture device such as a culture tank capacity of ○ ~ ○, an aspect ratio of ○ ~ ○, stirrer specifications of ○ ~ ○, a blade diameter/tank diameter ratio of ○ ~ ○, various device auxiliary equipment conditions, and various control device conditions. The device condition information may include a usage history, a usage status, and the like of the culture device in addition to the above.

The user inputs a device condition proposal from the input unit 6. Examples of the device condition proposal include, similarly to the device condition information, specifications of a culture device such as a culture tank capacity of ○ ~ ○, an aspect ratio of ○ ~ ○, stirrer specifications of ○ ~ ○, a blade diameter/tank diameter ratio of ○ ~ ○, various device auxiliary equipment conditions, and various control device conditions. The device condition proposal may include an outline, a function, and the like of the culture device in addition to the above.

The culture equipment matching program analyzes the device condition proposal and the device condition information, and determines a similarity therebetween. The culture equipment matching program (the extraction unit 43) extracts similar culture devices and lists the culture devices in descending order of similarity. Next, the culture equipment matching program (proposal output unit 44) proposes extracted and listed device conditions (culture tank or culture device), available times thereof, and the like. The listed device conditions can be presented, for example, as recommendation 1 of a device A, recommendation 2 of a device B, and recommendation 3 of a device C. For example, information such as a device number (HT00000001 or the like), a similarity (80% or the like), and a region name of the culture tank (city of ○○ or the like) can be presented to the user as proposal 1, proposal 2, proposal 3... or the like as a "list of optimal culture tank proposals for customer". For example, when proposal 1 is selected, as illustrated in FIG. 6, information on a past usage status such as "comment: it is often used for fermentation production using E. coli" and information such as "culture tank type: fermenter" can be obtained. Such information can be obtained from the device condition information stored in the storage unit 3.

### (Aspect 9)

In aspect 8, the input information to the input unit 6 preferably includes information on a strain, a cell line, or an organism species to be cultured, and a planned culture capacity. In this way, matching is performed with higher accuracy.

### (Aspect 10)

In this aspect, first, the culture experiment is conducted under the culture condition (first culture condition) of the culture experiment in the first scale culture equipment output previously (that is, this culture experiment is a second or subsequent culture experiment), and new experimental data including information on a change amount of a plurality of parameters indicating the state in the first scale culture equipment is obtained. The calculation unit 41 preferably performs the simulation based on the new experimental data and the second scale equipment information, and outputs, based on a result of the simulation, the condition range information that is information indicating a relationship between the second culture condition and the plurality of parameters. Further, the planning unit 42 preferably performs analysis based on the new experimental data and the condition range information, and newly outputs a culture condition (first culture condition) of the culture experiment in the first scale culture equipment.

In this aspect, since the culture experiment is conducted under the previously output first culture condition, the simulation is performed based on the experimental data, and the condition range information is output, the accuracy of the newly output first culture condition is improved. In this aspect, the processing can be repeated any number of times. Further, in this aspect, the processing can be repeated until the newly output first culture condition is sufficiently accurate, for example, until first culture conditions newly output in a plurality of times have substantially the same content. In this way, since a sufficient simulation is performed in the first scale, the accuracy of the first culture condition is further improved.

When the accuracy of the first culture condition is improved, the probability of success in culture in the second scale culture equipment can be improved. For example, the probability that constant productivity can be maintained can be improved. This leads to avoidance of a failure after transition to the second scale (that is, the constant productivity cannot be maintained) and reexamination from the first scale. Therefore, as a result, an examination time and an examination cost can be reduced.

### (Aspect 11)

In aspect 10, the calculation unit 41 preferably performs clustering analysis on the new experimental data before performing the simulation. Further, the planning unit 42 preferably outputs, based on a result of the clustering analysis, at least one of an optimal culture condition in the second culture condition and an important parameter to be focused on when scaling up a process of the culture target. The planning unit 42 preferably performs the simulation using at least one of the optimal culture condition and the important parameter. In this aspect, the calculation unit 41 performs the clustering analysis before performing the simulation, so that the planning unit 42 can more accurately extract and output the optimal culture condition in the second culture condition and the important parameter to be focused on when scaling up the process of the culture target. Therefore, the simulation result obtained by using the optimal culture condition and the important parameter has higher accuracy.

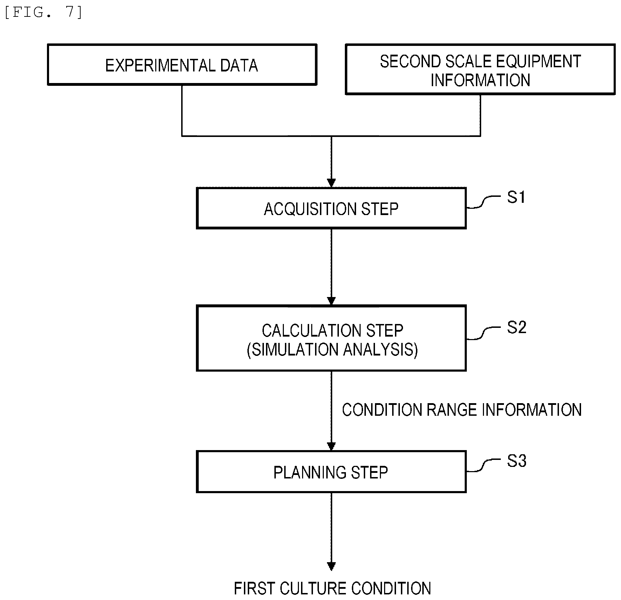

Next, a process development support method according to the embodiment will be described. FIG. 7 is a flowchart illustrating content of the process development support method according to the embodiment. As illustrated in FIG. 7, the process development support method according to the embodiment includes an acquisition step S1, a calculation step S2, and a planning step S3.

### (Acquisition Step S1)

In the acquisition step S1, experimental data and second scale equipment information are acquired. Since the experimental data and the second scale equipment information have already been described, a description thereof will be omitted. The experimental data and the second scale equipment information can be acquired from the storage unit 3, and may be acquired by being transmitted through the communication interface 2 or the input unit 6.

### (Calculation Step S2)

In the calculation step S2, based on the experimental data and the second scale equipment information, a simulation is performed regarding a change amount of same parameters same as the plurality of parameters in a case of culturing a culture target under a second culture condition in second scale culture equipment. In the calculation step S2, condition range information that is information indicating a relationship between the second culture condition and the plurality of parameters is output based on a result of the simulation. The calculation step S2 can be performed by the calculation unit 41.

### (Planning Step S3)

In the planning step S3, a culture condition of a culture experiment in the first scale culture equipment is output based on the experimental data and the condition range information. The planning step S3 can be performed by the planning unit 42.

### (Specific Example of Process Development Support Method)

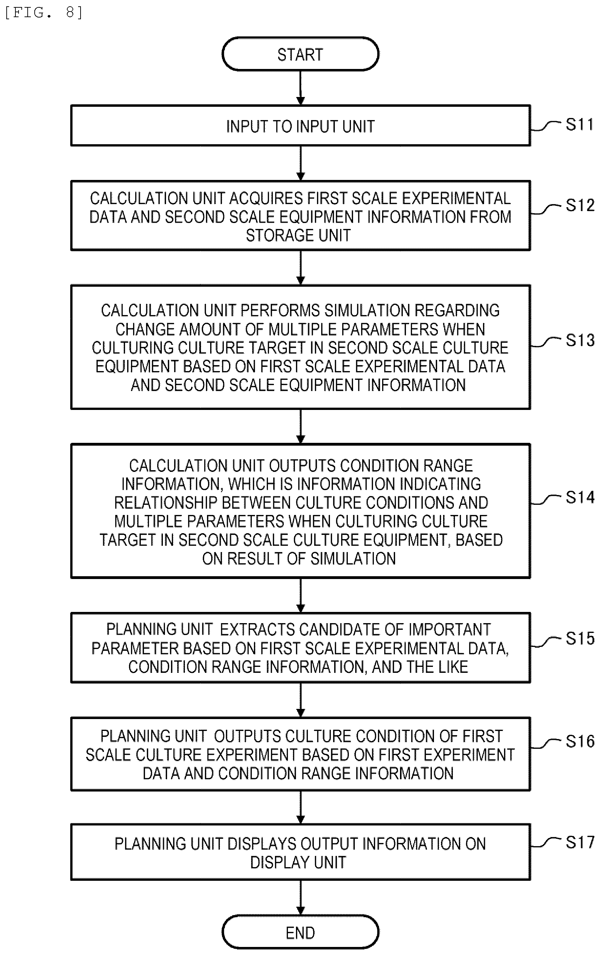

FIG. 8 is a flowchart illustrating a specific example of the process development support method according to the embodiment. As illustrated in FIG. 8, information on at least one of specifications, an outline, and a function of a culture tank or a culture device corresponding to second scale culture equipment desired to be used by a user is received by the input unit 6 (step S11) and stored in the storage unit 3 of the process development support system S. The user inputs, to the input unit 6, experimental data including information on a change amount of a plurality of parameters indicating a state in first scale culture equipment when a culture target is subjected to a culture experiment under a first culture condition in the first scale culture equipment (step S11), and stores the experimental data in the storage unit 3 of the process development support system S. Next, the calculation unit 41 acquires the first scale experimental data and second scale equipment information from the storage unit 3 (step S12) .

Next, based on the first scale experimental data and the second scale equipment information, the calculation unit 41 performs a simulation regarding a change amount of the plurality of parameters when culturing the culture target in the second scale culture equipment (step S13) .

Next, based on a result of the simulation, the calculation unit 41 outputs condition range information that is information indicating a relationship between a culture condition (second culture condition) and the plurality of parameters when culturing the culture target in the second scale culture equipment (step S14). Next, the planning unit 42 extracts a candidate of an important parameter based on the first scale experimental data, the condition range information, and the like (step S15).

Next, the planning unit 42 outputs a new culture condition (new first culture condition) of the culture experiment in the first scale based on first experimental data and the condition range information (step S16). Here, regarding the new culture condition of the culture experiment, the planning unit 42 may calculate an important parameter in process development of scale-up from the first scale culture equipment to the second scale culture equipment based on the condition range information and the experimental data, and output the new culture condition of the culture experiment based on information on the important parameter. In this way, since the new culture condition of the culture experiment is output based on the information on the important parameter, the culture experiment in the first scale culture equipment conducted on the basis thereof can obtain a more suitable result in consideration of a specific culture condition on the second scale. The planning unit 42 displays the output information on a display unit (output device 7) (step S17).

### (Description of Effects and Alternative Configurations)

The planning unit 42 that outputs a culture condition of a culture experiment to be examined on a first scale can not only set a condition range but also propose and recommend a condition of the culture equipment for acquiring the culture condition. For example, when there is a substrate concentration distribution, it is possible to propose a device capable of reproducing the concentration distribution. Furthermore, a shape of a stirring blade or the like is also proposed.

The process development support system S and the process development support method can propose and recommend a condition of second scale culture equipment required to minimize items with large distributions and parameters with high influence in consideration of the important parameter and the condition range information. For example, the process development support system S and the process development support method can propose and recommend a shape of a culture tank, a stirring blade used in the culture tank, a stirring condition, an air diffuser condition for a bubble diameter, and the like. Further, the process development support system S and the process development support method can propose the information on the culture equipment to be recommended from the equipment information stored in the storage unit 3 or the like. In the case of recommendation of the culture equipment, an installation location, an operation status, a use history, and the like may be indicated in addition to equipment information and specifications.

By such means, it is possible to understand the specific culture conditions on the second scale, and support the creation of an experimental plan on the first scale while considering the culture conditions. Accordingly, even when experimental data information on the second scale is not sufficiently obtained, it is possible to reduce an uncertain condition that cannot be reproduced or an unassumed constraint condition, and it is possible to prevent a decrease in productivity on the second scale due to the equipment. Finally, it is preferable to perform a state simulation in the second scale using the data about the result of the first scale experiment, and it is preferable to confirm whether the culture is within the assumption, that is, to confirm the productivity and the like by conducting the culture experiment in the second scale culture equipment. In data acquisition examined under proposed culture conditions, the example of the first scale culture equipment is shown, but it is not necessarily required to conduct the experiment in the first scale. That is, the proposed culture conditions may be examined using culture equipment of a scale other than the first scale. In this case, when outputting a recommended experimental condition of the planning unit 42, information on available scales may be output together. Application Examples

### (Example of Microorganism A)

Here, an example in which the process development support system S and the process development support method described according to the embodiment are applied to a process of performing batch culture of microorganisms A using saccharides as raw materials to generate organic compounds will be described.

In this application example, the following were prepared as experimental data. First, a culture condition in a culture tank having a volume of 10 L as a first scale and experimental data were used. As the experimental data, in addition to information on the microorganism A, a temperature, a pH, a DO concentration, a medium concentration as an addition condition, a liquid amount, a viscosity, and the like were prepared. In addition, conditions of an aeration amount, a stirring rotation speed, and a pressure were prepared as control indexes for adjusting an environment. Next, equipment information of culture equipment having a volume of 1000 L as a second scale assuming culture was prepared. As equipment conditions, an aspect ratio of the culture tank, an aerator, a stirrer, pH adjustment equipment, and a control device that controlling a DO concentration, and specifications thereof were prepared.

Based on these, a change amount of parameters of culture conditions in the culture tank having a volume of 1000 L was analyzed using fluid analysis software. Changes in temperature, pH, DO concentration, medium concentration, additive concentration, and the like were analyzed to clarify various concentration distributions. In this application example, content focusing on the DO concentration will be described. The distribution of the DO concentration in a steady state was found in consideration of the degree of dissolution of oxygen from a rotation condition and an aeration condition of the stirrer, a viscosity condition, the temperature, and the like.

It was confirmed that there was a variation in the DO concentration under the rotation condition and the aeration condition of the stirrer based on the initial conditions. Here, it was confirmed that the DO concentration in the culture tank had a distribution from 0.5 mg/L to 8 mg/L. It was also found that a shear stress increased when the rotation speed of the stirrer was increased. On the other hand, it was also found that a DCO₂ concentration increased when the DO concentration was low, and the relationship between the culture conditions and the plurality of parameters was shown.

From the first scale experimental data, it was confirmed that the DO concentration in the culture condition test in the culture tank having a capacity of 10 L was 5 mg/L or more as a sensor representative value at one point, but since there was no condition of the range of the DO concentration, the DO concentration was set as an important parameter. Since it was found that the DCO₂ concentration and the shear stress, which had almost no data in the first scale experimental data, had a certain range, the DCO₂ concentration and the shear stress were set as important parameters.

The planning unit 42 specified and output a culture condition, in which the DO concentration included a range of 0.5 mg/L to 8 mg/L, as an experimental condition. The planning unit 42 output a culture condition range that satisfied the conditions of the similarly extracted DCO₂ concentration and shear stress. The planning unit 42 proposed culture equipment capable of changing the DO concentration using nitrogen gas and oxygen gas, and output the culture equipment to the display unit.

Based on this, the experimenter carried out an organic compound productivity experiment at a DO concentration of 0.5 mg/L to 8 mg/L in a culture tank having a capacity of 500 mL. As a result, it was confirmed that the productivity decreased significantly with the DO of less than 1 mg/L, more specifically, 0.5 mg/L.

An experiment for confirming whether the DCO₂ concentration and the shear stress were affected was conducted under the conditions of the second scale culture equipment in which the DO concentration was 1 mg/L or more. As a result, it was confirmed that the production of organic compounds was not significantly affected.

As a result of performing the simulation again under these conditions, a sufficient state was confirmed in the culture tank having a capacity of 1000 L. In view of the above, optimal culture conditions in the culture tank having a capacity of 1000 L could be found.

Although the process development support system S and the process development support method according to the invention have been described in detail above through embodiments and application examples, the invention is not limited to the above-described embodiments and application examples, and various modifications are included. For example, the above-described embodiments have been described in detail to facilitate understanding of the invention, and the invention is not necessarily limited to those including all the configurations described above. A part of a configuration of a certain embodiment can be replaced with a configuration of another embodiment, and a configuration of another embodiment can be added to a configuration of a certain embodiment. Another configuration can be added to, deleted from, or replaced with a part of a configuration of each embodiment.

### Reference Signs List

S: process development support system
1: management server
2: communication interface
3: storage unit
4: control unit
41: calculation unit
42: planning unit
43: extraction unit
44: proposal output unit
5: memory
6: input unit
7: output device
2A1: second scale analysis distribution range
2A2: initial first scale experimental range
2B1: first scale input experimental condition
2B2: range
2B3: second scale analysis distribution range
2C1: first scale Input experimental condition
2C2: range
2D1: first scale Input experimental condition
2D2: first scale initial experimental range
2E1: initial first scale experimental range
2E2: range
2E3: first scale Input experimental condition
2E4: range
2E5: range
2E6: range
2E7: culture condition range

## Claims

1. A process development support system comprising:
a storage unit configured to store experimental data including information on a change amount of a plurality of parameters indicating a state in first scale culture equipment in a case where a culture target is subjected to a culture experiment under a first culture condition in the first scale culture equipment, and second scale equipment information that is information on an equipment condition including at least shape information of second scale culture equipment larger than the first scale culture equipment;
a calculation unit configured to perform, based on the experimental data and the second scale equipment information, a simulation related to a change amount of parameters same as the plurality of parameters in a case where the culture target is cultured under a second culture condition in the second scale culture equipment, and output, based on a result of the simulation, condition range information that is information indicating a relationship between the second culture condition and the plurality of parameters; and
a planning unit configured to output a culture condition of the culture experiment in the first scale culture equipment based on the experimental data and the condition range information.

2. The process development support system according to claim 1, wherein
the planning unit specifies an important parameter in process development of scale-up from the first scale culture equipment to the second scale culture equipment based on the condition range information and the experimental data, and outputs a culture condition of the first scale culture experiment based on information on the important parameter.

3. The process development support system according to claim 1, wherein
the first culture condition includes information on set values of items of a plurality of operation conditions for controlling the first scale culture equipment, and
the planning unit outputs, based on the experimental data and the condition range information, a range in which the set values in the culture experiment are changed.

4. The process development support system according to claim 1, wherein
the planning unit outputs information on a magnitude of influence in a case of culturing the culture target in the second scale culture equipment, based on information on the plurality of parameters included in the experimental data and information on the plurality of parameters included in the condition range information.

5. The process development support system according to claim 1, wherein
the storage unit stores first equipment information regarding a plurality of candidates for the first scale culture equipment, and
the planning unit outputs information on culture equipment to be used for the culture experiment among the first equipment information.

6. The process development support system according to claim 2, wherein
the storage unit stores second equipment information regarding a plurality of candidates for the second scale culture equipment, and
the planning unit outputs information on a candidate for culture equipment to be used as the second scale culture equipment, based on information on a range and a behavior of parameters same as the plurality of parameters, the information on the important parameter, and the second equipment information.

7. The process development support system according to claim 1, wherein
the first culture condition is at least one of a viscosity, a concentration of various components, a temperature, a pH, a dissolved oxygen concentration, a stirring rotation speed, an aeration amount, or a pressure.

8. The process development support system according to claim 1, wherein
the equipment condition is at least one of an aspect ratio of a culture tank, an aerator, a stirrer, pH adjustment equipment, a control device that controls a dissolved oxygen concentration, and specifications thereof.

9. The process development support system according to claim 1, wherein
the calculation unit simulates, based on the second culture condition and the second scale equipment information, behaviors of various parameters when culture is performed in the second scale culture equipment, and
the various parameters are at least one of a shear force or a dissolved oxygen concentration.

10. The process development support system according to claim 1, further comprising:
a database configured to store information on candidates for the second scale culture equipment and information on specifications and usage of the candidates;
an input unit configured to receive a condition of the second scale culture equipment;
an extraction unit configured to extract, from among the candidates for the second scale culture equipment, one or more candidates for the culture equipment that are related to the condition received by the input unit, based on information received by the input unit and the information stored in the database; and
a proposal output unit configured to propose the candidates for the culture equipment extracted by the extraction unit and information regarding usage of the candidates.

11. The process development support system according to claim 10, wherein
input information to the input unit includes information on a strain, a cell line, or an organism species to be cultured, and a planned culture capacity.

12. The process development support system according to claim 1, wherein
a culture experiment is conducted under the culture condition of the culture experiment in the first scale culture equipment to obtain new experimental data including information on a change amount of a plurality of parameters indicating a state in the first scale culture equipment,
the calculation unit performs the simulation based on the new experimental data and the second scale equipment information and outputs, based on a result of the simulation, condition range information that is information indicating a relationship between the second culture condition and the plurality of parameters, and
the planning unit performs analysis based on the new experimental data and the condition range information and newly outputs a culture condition of the culture experiment in the first scale culture equipment.

13. The process development support system according to claim 12, wherein
the calculation unit performs clustering analysis on the new experimental data before performing the simulation, and
the planning unit outputs, based on a result of the clustering analysis, at least one of an optimal culture condition in the second culture condition and an important parameter to be focused on when scaling up a process of the culture target, and performs the simulation using at least one of the optimal culture condition and the important parameter.

14. A process development support method comprising:
an acquisition step of acquiring experimental data including information on a change amount of a plurality of parameters indicating a state in first scale culture equipment in a case where a culture target is subjected to a culture experiment under a first culture condition in the first scale culture equipment, and equipment information of a second scale that is information on an equipment condition including at least shape information of culture equipment of the second scale larger than the first scale culture equipment;
a calculation step of performing, based on the experimental data and the second scale equipment information, a simulation related to a change amount of parameters same as the plurality of parameters in a case where the culture target is cultured under a second culture condition in the second scale culture equipment, and outputting, based on a result of the simulation, condition range information that is information indicating a relationship between the second culture condition and the plurality of parameters; and
a planning step of outputting a culture condition of the culture experiment in the first scale culture equipment based on the experimental data and the condition range information.
